# EUROPEAN PATENT APPLICATION

(11) **EP 3 977 874 A1**
(43) Date of publication of application: **06.04.2022**
(21) Application number: 20200048.5
(22) Date of filing: 05.10.2020
(51) Int. Cl.: A24F 40/51, A24F 40/65, A61M 15/06, H04B 10/114

(54) **VAPING DEVICE HAVING AN OPTICAL SENSOR FOR DATA RECEPTION**

(71) Applicant: JT International S.A., 1202 Geneva (CH)
(72) Inventor: VERLAAN, Theodorus, 1206 GENEVE (CH)
(74) Representative: Santarelli

(57) **Abstract**

The present invention thus relates to a vaping device comprising a body (4), an atomizer (2) for generating an inhalable vapor, a microcontroller accommodated into the body for controlling the atomizer and a user interface for bilateral communication of data with the microcontroller. The user interface comprises a first optical sensor for data reception (7), such as an infrared sensor like a photodiode or a phototransistor, connected to the microcontroller and able to receive light signals from an external communicating apparatus having a light source, to convert the light signals into electrical signals and to transmit the electrical signals to the microcontroller. Such a first optical sensor for data reception constitute means for communication between an ancillary device such as a smartphone, and the vaping device, that are cheap and resistant to attacks and that enable a user to send program data to the e-vaping device from a smartphone in a easy and reliable way.

## Description

### Field of the invention

The present disclosure relates to a vaping device such as an e-cigarette which creates an inhalable vapor by electronically heating an e-liquid, or such as a vapor tobacco device which contains tobacco and/or other suitable solid substrates that are heated, though not burnt, to create an inhalable vapor.

A vaping device generally include: a box containing a microcontroller; a user interface for bilateral communication with the microcontroller; a battery; an atomizer containing a chamber for receiving either an e-liquid or a solid stick of a substance suitable to be heated to generate an inhalable vapor (for example a tobacco-containing stick) and an electrical heater powered by the battery and controlled by the microcontroller for heating the e-liquid or the stick by conduction, convection and/or radiation.

The vaping experience depends on various parameters such as the maximum power delivered to the heater by the battery, the maximum temperature of the heater, the ramp-up (rise time to reach the desired maximum temperature or power), the inlet air flow, etc. These parameters may be set differently according to the e-liquid or the solid substrate to be vaporized and/or according to the used material (for example, various coils in Ni, NiFe, stainless steel, Ti, etc. may be used as the heater in an e-cigarette) and/or according to the user's mood and wishes. The vaping device may be controlled according to various modes, for example a power (watt) mode, where the microcontroller controls the power delivered to the heater according to set point values of power, or a temperature mode, where the microcontroller controls the temperature of the heater and adjust the power delivered to the heater according to the current temperature of the heater to reach set point values of temperature.

### Background of the invention

All these parameters should be likely to be chosen by the user of the vaping device and may be input as setting or program data in the microcontroller via the user interface to program the vaping device.

In brief, providing data, in particular program data, to a vaping device is desirable.

To this end, the user interface of the known vaping devices usually includes a screen to display a parameter value and setup buttons "+" and "-" to choose a desired value for the parameter by increasing or decreasing the displayed value. The setting of a vaping device in this way is burdensome.

GB2533137 discloses an e-cigarette comprising: an electrical heater for vaporizing a liquid to form an aerosol for inhalation by a user and a Bluetooth interface for providing wireless communications with an auxiliary device such as a smartphone; wherein the Bluetooth interface is configured to use the heater as an antenna for the wireless communications.

The data connection established between the smartphone and the e-cigarette allows for full data exchange between the devices. This is however cost intensive, as the e-cigarette needs to be provided with expensive communications means, controls, battery power etc. Also, some consumers are worried about data security and unneeded data connections are perceived negatively.

The invention aims to provide for a method and means for communication between a vaping device and an ancillary device, that are cheap to implement, functional, and resistant to attacks, having therefore a high acceptance factor by users (with respect to data privacy concerns).

### Summary of the invention

The present invention thus relates to a vaping device comprising a body, an atomizer for generating an inhalable vapor, a microcontroller accommodated into the body for controlling the atomizer, and a user interface for bilateral communication of data with the microcontroller, characterized in that the user interface comprises a first optical sensor for data reception, connected to the microcontroller and able to receive light signals from an external communicating apparatus, to convert the light signals into electrical signals and to transmit the electrical signals to the microcontroller.

The first optical sensor for data reception may include or be constituted of an infrared sensor, in particular a photodiode or a phototransistor.

Optical sensors such as photodiodes and phototransistors are cheap electronic components. Moreover, a light signal transmission is very difficult for an attacker to intercept effectively.

The data transmitted might be, for example, program data to program the device, for example to set a heater profile or to input custom user settings.

Furthermore, an unlock code may be sent to the vaping device. The received code is compared to a code stored in a memory of the microcontroller, and the vaping device is unlocked when the correct code is received. This provides a high level of security as the data transmission (lights signals) is very difficult to intercept by an attacker.

The external communicating apparatus which sends the data to the vaping device is provided with a light source and a control section that is able to convert the data to be sent into a coded light pulse signal.

The external communicating apparatus may be a smartphone with a light source.

The phone is provided with an application that can receive input from the user. The application converts the settings, program data or other data previously input by the user, using a data protocol, and sends out the corresponding light signals after a command by the user.

Preferably, the body of the vaping device has a recess where the first optical sensor for data reception is accommodated, the recess and the first optical sensor for data reception being covered by a layer of translucent or semi-translucent material, an external face of this layer being flush with an external face of the body.

Optionally, the first optical sensor for data reception is hidden by said translucent or semi-translucent layer but is associated with a visual indication for locating the first optical sensor for data reception. In a potential embodiment, the visual indication is a painted print on the body surrounding the first optical sensor for data reception.

If needed, the first optical sensor for data reception may be covered by a suitable light guide configured to focus light toward the first optical sensor for data reception to improve light detection and make sure that the sensor does not need to be directly positioned in line with a main light emitting direction of the external communicating apparatus.

Any suitable data protocol may be used. Preferably, the microcontroller is configured to treat the electrical signals emitted by the first optical sensor for data reception according to a data protocol chosen among the Morse code, a binary code, etc. The Morse code distinguishes between long and short signals, as well as no signal. Binary codes distinguish only between signal/no signal over time.

Preferably, the used data protocol is based on light pulses having a frequency of more than 50 Hz. Such pulses are faster than those likely to be captured by typical digital cameras. This improves resistance to attacks.

Preferably, the light signals are received in batch, each batch containing a call signal indicating the beginning of the batch, program data signals to program the vaping device, a checksum signal indicating the end of the batch, the checksum signal being preceded by a control signal.

In a preferred embodiment, the vaping device comprises an ambient light sensor connected to the microcontroller, the microcontroller being configured to adjust the sensitivity of the first optical sensor for data reception according to the signal received from the ambient light sensor. In that way, the sensitivity of the first optical sensor for data reception is adjusted to ambient light conditions, inducing a more accurate data reception.

To this end, the microcontroller may include a fuzzy logic circuit which controls a (digital) potentiometer connected to the first optical sensor for data reception, resulting in a stable voltage output independent of the ambient light intensity.

Alternatively, the ambient light sensor is the first optical sensor for data reception.

If so, the ambient measurement may be taken either before a data transmission is started, or just after a call signal has been received. In this second case, the detection of a call signal by the microcontroller may involve a brief pause in signal transmission to allow for an ambient light measurement to be taken by the first optical sensor for data reception.

Optionally, the vaping device includes a second optical sensor for data reception, the first optical sensor for data reception and the second optical sensor for data reception being located on opposite sides of the body, the microcontroller being configured to determine which one of the first or second optical sensor for data reception currently receives the highest light intensity and to use this sensor for the current data reception, the other sensor being then temporarily used for ambient light measurements.

The invention also concerns a method for programming a vaping device which comprises a body, an atomizer for generating an inhalable vapor, a microcontroller accommodated into the body for controlling the atomizer and a user interface for bilateral communication of data with the microcontroller. This method is characterized in that it comprises:
- using a vaping device whose user interface comprises a first optical sensor for data reception, connected to the microcontroller and able to convert light signals into electrical signals and to transmit the electrical signals to the microcontroller, and using a smartphone having a light source for emitting light signals,
- defining vaping program data in an application implemented in the smartphone,
- converting these vaping program data into light signals and causing the light source of the smartphone to emit these light signals in direction to the vaping device,
- reception of these light signals by the first optical sensor for data reception of the vaping device and treatment by the microcontroller of the corresponding electrical signals transmitted by the first optical sensor for data reception.

The invention further concerns a system for programming a vaping device, the system comprising:
- the vaping device itself, which vaping device comprises a body, an atomizer for generating an inhalable vapor, a microcontroller accommodated into the body for controlling the atomizer and a user interface for bilateral communication of data with the microcontroller.
- and a smartphone.

The system is characterized in that the vaping device comprises a first optical sensor for data reception, connected to the microcontroller and able to convert light signals into electrical signals and to transmit the electrical signals to the microcontroller, and in that the smartphone comprises a light source for emitting light signals and an application for defining vaping program data and for converting these vaping program data into light signals.

The invention finally proposes an application for smartphone characterized in that it comprises instructions which, when loaded and run on the smartphone of the above described system, causes the system to perform the above-described method.

### Brief description of the drawings

Other particularities and advantages of the invention will also emerge from the following description.

In the accompanying drawings, given by way of non-limiting examples:
- Figure 1 represents a schematic side view of a vaping device according to a first embodiment of the invention;
- Figure 2 is a schematic cross-section in a longitudinal plan of the first embodiment of figure 1;
- Figure 3 is a schematic side view of a vaping device according to a second embodiment of the invention;
- Figure 4 is a schematic cross-section in a longitudinal plan of the second embodiment of figure 3;
- Figure 5 represents a schematic side view of a vaping device according to a third embodiment of the invention.

### Detailed Description

All the vaping devices illustrated in the annexed drawings include, from a lower end to an upper end:
- a battery module 1 having a body 4 which accommodates a battery 5 and a number of electronic components including a microcontroller 6 in the form of a main printed circuit board assembly; the microcontroller 6 is connected to the battery 5 and powered by the latter in a conventional way (not shown); the battery module may further include, at its lower end, connecting means (not shown) for connecting the battery to a charger (not shown) supplied with electricity by a suitable transformer or by a USB (Universal Serial Bus) socket;
- an atomizer 2 where a substance, either in liquid state or in a solid state, is heated to be converted into particles small and light enough to be carried in the air for inhalation by a user, the atomizer accommodating an electrical heater powered by the battery and controlled by the microcontroller; the atomiser 2 might be an open-tank part which can be refilled with a volume of the substance to be atomized or a consumable cartridge which is entirely changed when it is empty of substance to be atomized;
- and a mouthpiece 3 where the user places his mouth to vape.

The vaping devices also comprise a user interface including buttons and indicators such as a power button located on the battery module (not shown in the drawings) to turn on or off the vaping device and a light indicator associated with the power button for indicating whether the device is on or off. The user interface may also include a screen (not shown) for displaying information for the attention of the user.

According to the invention, the user interface further comprises a first optical sensor for data reception 7 able to receive light signals from an external device such as a smartphone and to convert the light signals into electrical signals. The first optical sensor for data reception 7 is connected to the microcontroller 6 such that it can transmit electrical signals to the microcontroller 6. This connection is achieved in a conventional way (not shown).

The first optical sensor for data reception 7 is used to communicate data to the microcontroller 6 from an external device such as a smartphone.

These data might be vaping program data such as values of various vaping parameters including the maximum temperature to which the substance to vape is to be heated, the ramp-up, the inlet air flow, a time period at the end of which the vaping device is to be turned off, etc., these values being preferred values defining a consumer profile or temporary values to be applied to the next use of the vaping device.

As it is easier to program such vaping parameters by the way of an application implemented in a smartphone rather than directly by the way of the user interface of the vaping device, it is desirable to provide the vaping device with means for receiving data from a smartphone (or other external device).

The invention proposes to provide the vaping device with a first optical sensor for data reception 7 to this end. The first optical sensor for data reception 7 might be of a photodiode or phototransistor 7 or, more generally speaking an infrared sensor.

Such an optical sensor is cheap, easy to implement and resistant to attack, contrary to a Bluetooth connection for example.

This photodiode 7 is accommodated in the body 4 of the battery module 1.

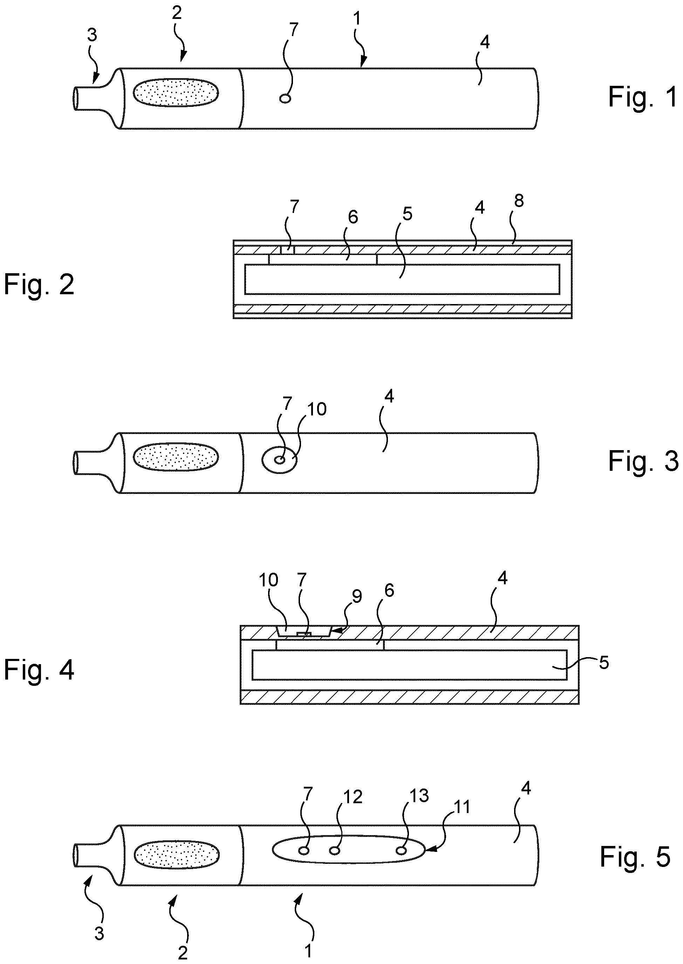

In a first embodiment represented at figures 1 and 2, a hole corresponding to the size of the photodiode 7 is provided in the body 4. The photodiode 7 is fitted into this hole and fastened therein by any appropriate mean such as an adhesive substance. Moreover, an even layer 8 of a translucent or semi-translucent material covers the entire body 4 of the battery module.

The positioning of the photodiode 7 on the battery module is not essential, but preferably the photodiode 7 is placed in the vicinity of an upper end of the body 4 so as to make it easier for the user to hold the device by the lower end while using the smartphone to irradiate the sensor.

In a second embodiment represented at figures 3 and 4, a recess 9 larger than the photodiode 7 is provided in the body 4 in the vicinity of the upper end of the body. The photodiode 7 is accommodated in this recess 9 and the recess is filled with a layer of translucent or semi-translucent material 10 to secure the photodiode in the recess. The translucent layer 9 being flush with the external peripheral face of the body.

In a third embodiment represented at figure 5, a larger recess 11 is provided for accommodating both the photodiode 7 used as the first sensor for data reception and other sensors and/or indicators.

For example, the recess 11 may receive a second optical sensor 12 for ambient light measurements. This ambient light sensor 12 is connected to the microcontroller 6, which microcontroller is configured to adjust the sensitivity of the first optical sensor for data reception 7 according to the signal received from the ambient light sensor 12.

The recess 11 may further receive one or more light indicator(s) 13 for indication of the heating status, the battery status, etc.

Again, the recess 11 is filled with a layer of translucent or semi-translucent material which is flush with the external peripheral face of the body 4.

In all these embodiments the microcontroller 6 is configured to treat the electrical signals emitted by the first optical sensor for data reception (or photodiode) 7 according to a data protocol chosen among the Morse code (which distinguishes between long and short signals as well as no signal), a binary based code (which distinguishes only between signal and no signal over time).

For initiating the transmission of a batch of data between the smartphone and the vaping device, a call signal is first emitted by the smartphone. Then program data signals are sent, followed by a control signal and a checksum signal which indicates the end of the batch.

As previously explained, the embodiment illustrated at figures 5 includes an ambient light sensor 12, which enables the microcontroller 6 to take into account the current ambient light while treating the electrical signals of the optical sensor for data reception; in other words, the ambient light sensor 12 makes it possible to adjust the sensitivity of the optical sensor for data reception according to the current ambient light.

Alternatively, the first sensor for data reception may also operate as an ambient light sensor.

In this case, the detection of a call signal received by the optical sensor for data reception 7 from the smartphone may involve a brief pause in signal transmission to allow for an ambient light measurement to be taken by the first optical sensor for data reception.

In a fourth example (not represented) the user interface may comprise two optical sensors for data reception, namely the first optical sensor for data reception 7 and a second optical sensor for data reception which might be identical to the first optical sensor for data reception but which is located on an opposite side of the body 4. The microcontroller is then advantageously configured to determine which one of the first or second optical sensor for data reception receives the highest light intensity and to use this sensor for the data reception if a call signal is received. The other sensor is then used for ambient light measurements in order to adjust the sensitivity of the sensor used for the data reception.

The invention extends to all the alternative embodiments that are covered by the appended claims.

## Claims

1. A vaping device comprising a body (4), an atomizer (2) for generating an inhalable vapor, a microcontroller (6) accommodated into the body for controlling the atomizer and a user interface for bilateral communication of data with the microcontroller, **characterized in that** the user interface comprises a first optical sensor for data reception (7), connected to the microcontroller (6) and able to receive light signals from an external communicating apparatus having a light source, to convert the light signals into electrical signals and to transmit the electrical signals to the microcontroller.

2. The vaping device according to claim 1, wherein the body has a recess (9, 11) where the first optical sensor for data reception is accommodated, the recess and the first optical sensor for data reception being covered by a layer of translucent or semi-translucent material (10), this layer being flush with an external face of the body.

3. The vaping device according to any one of claims 1 and 2, wherein the first optical sensor for data reception (7) is hidden by said translucent or semi-translucent layer but is associated with a visual indication for locating the first optical sensor for data reception.

4. The vaping device according to any one of claims 1 to 3, wherein the first optical sensor for data reception (7) is covered by a suitable light guide configured to focus light toward the first optical sensor for data reception.

5. The vaping device according to any one of claims 1 to 4, wherein the first optical sensor for data reception includes a photodiode or a phototransistor (7).

6. The vaping device according to any one of claims 1 to 5, wherein the microcontroller (6) is configured to treat the electrical signals emitted by the first optical sensor for data reception (7) according to a data protocol chosen among the Morse code, a binary based code.

7. The vaping device according to claim 6, wherein the protocol is based on light pulses having a frequency of more than 50 Hz.

8. The vaping device according to any one of claims 1 to 7, wherein the vaping device comprises an ambient light sensor (7; 12) connected to the microcontroller (6), the microcontroller being configured to adjust the sensitivity of the first optical sensor for data reception (7) according to the signal received from the ambient light sensor.

9. The vaping device according to any one of claims 1 to 8, wherein the microcontroller includes a fuzzy logic circuit which controls a potentiometer connected to the first optical sensor for data reception, resulting in a stable voltage output independent of the ambient light intensity.

10. The vaping device according to claim 9, wherein the ambient light sensor is the first optical sensor for data reception (7).

11. The vaping device according to any one of claim 1 to 10, wherein the user interface includes a second optical sensor for data reception, the first optical sensor for data reception (7) and the second optical sensor for data reception being located on opposite sides of the body (4), the microcontroller (6) being configured to determine which one of the first or second optical sensor for data reception currently receives the highest light intensity and to use this sensor for the current data reception, the other sensor being then temporary used for ambient light measurements.

12. A method for programming a vaping device which comprises a body (4), an atomizer (2) for generating an inhalable vapor, a microcontroller (6) accommodated into the body (4) for controlling the atomizer and a user interface for bilateral communication of data with the microcontroller, the method being **characterized in that** it comprises:
- using a vaping device whose user interface comprises a first optical sensor for data reception (7), connected to the microcontroller (6) and able to convert light signals into electrical signals and to transmit the electrical signals to the microcontroller, and using a smartphone having a light source for emitting light signals,
- defining vaping program data in an application implemented in the smartphone,
- converting these vaping program data into light signals and causing the light source of the smartphone to emit these light signals in direction of the vaping device,
- reception of these light signals by the first optical sensor for data reception (7) of the vaping device and treatment by the microcontroller (6) of the corresponding electrical signals transmitted by the first optical sensor for data reception (7).

13. The method according to claim 12, wherein the light signals are received in batch, each batch containing a call signal indicating the beginning of the batch, program data signals to program the vaping device, a checksum signal indicating the end of the batch, the checksum signal being preceded by a control signal.

14. A system for programming a vaping device which comprises a body (4), an atomizer (2) for generating an inhalable vapor, a microcontroller (6) accommodated into the body (4) for controlling the atomizer and a user interface for bilateral communication of data with the microcontroller, the system further comprising a smartphone, **characterized in that** the vaping device comprises a first optical sensor for data reception (7), connected to the microcontroller (6) and able to convert light signals into electrical signals and to transmit the electrical signals to the microcontroller (6), and **in that** the smartphone comprises a light source for emitting light signals and an application for defining vaping program data and for converting these vaping program data into light signals.

15. An application for smartphone **characterized in that** it comprises instructions which, when loaded and run on the smartphone of the system of claim 14, causes the system to perform the method of claim 12 or 13.
